# EUROPÄISCHE PATENTSCHRIFT

(11) **EP 3 873 553 B1**
(45) Veröffentlichungstag und Bekanntmachung des Hinweises auf die Patenterteilung: **04.09.2024**
(21) Anmeldenummer: 19798238.2
(22) Anmeldetag: 31.10.2019
(51) Int. Cl.: A61M 60/873, A61M 60/178, A61M 60/216, A61M 60/508, A61M 60/592, H02J 50/10, H04W 4/80

(54) **SYSTEM UND VERFAHREN ZUR STEUERUNG EINES HERZUNTERSTÜTZUNGSSYSTEMS**
SYSTEM AND METHOD FOR CONTROLLING A CARDIAC ASSISTANCE SYSTEM
SYSTÈME ET PROCÉDÉ DE COMMANDE D'UN DISPOSITIF D'ASSISTANCE VENTRICULAIRE

(30) Priorität: 02.11.2018 DE 102018218770
(43) Veröffentlichungstag der Anmeldung: 08.09.2021
(73) Patentinhaber: Kardion GmbH, 70376 Stuttgart (DE)
(72) Erfinder: WENNING, Leon, 76137 Karlsruhe (DE); SCHELLENBERG, Inga, 70376 Stuttgart (DE); HUEBNER, Annika, 71522 Backnang (DE)
(74) Vertreter: Pfiz, Thomas
(86) Internationale Anmeldenummer: PCT/EP2019/079905
(87) Internationale Veröffentlichungsnummer: WO 2020/089429

(56) Entgegenhaltungen:
- US-A1- 2012 022 645
- US-A1- 2016 022 889
- US-A1- 2016 095 968
- US-A1- 2017 136 164
- US-A1- 2018 256 796
- US-A1- 2018 256 800

## Beschreibung

Die Erfindung betrifft ein System zur Steuerung eines Herzunterstützungssystems mit einem Steuergerät, und ein Herzunterstützungssystem..

Bei modernen Herzunterstützungssystemen, auch VAD für "ventricular assist device" oder ventrikuläres Unterstützungssystem genannt, wird üblicherweise eine Pumpe zur Unterstützung der Herzfunktion an oder in das Herz implantiert, welche über ein Steuergerät geregelt wird. Dabei kann neben oder statt einem ebenfalls implantierten Steuergerät ein sogenanntes extrakorporales Steuergerät vorgesehen sein, welches sich außerhalb des Körpers des Patienten befindet, um eine externe Steuerung zu ermöglichen.

Beispielsweise ist aus US 2005/0107658 A1 ein Herzunterstützungssystem bekannt, welches sowohl ein implantiertes Steuergerät als auch ein extrakorporales Steuergerät umfasst. Das extrakorporale Steuergerät ist dabei drahtlos mit dem implantierten Steuergerät verbunden und dient vor allem zum bidirektionalen Datenaustausch.

Aus der US2016/0095968 A1 ist ein Herzunterstützungssystem mit einer ersten Blutpumpensteuerung als erstes extrakorporales Steuergerät und einer zweiten redundanten Blutpumpensteuerung als zweites extrakorporales Steuergerät bekannt, wobei das erste Steuergerät mit einem Kabel zur Kommunikation und Energieübertragung mit der Herzunterstützungssystem verbunden ist. Das Herzunterstützungssystem wird über eine externe Energiequelle bzw. Batterien mit Energie versorgt.

Ausgehend hiervon liegt der Erfindung die Aufgabe zugrunde, die bekannten Systeme weiter zu verbessern und insbesondere eine einfache und schnelle Informationserfassung sowie eine darauf abgestimmte Ansteuerung zu ermöglichen, die Handhabung zu erleichtern, die Nutzerfreundlichkeit zu erhöhen und einen zuverlässigen und sicheren Betrieb zu gewährleisten.

Vor diesem Hintergrund betrifft die Erfindung ein System zur Steuerung eines Herzunterstützungssystems, umfassend ein erstes extrakorporales Steuergerät und ein mit dem ersten Steuergerät und/oder mit dem Herzunterstützungssystem drahtlos verbundenes zweites extrakorporales Steuergerät, wobei das erste Steuergerät mit einer ersten Spule zur Kommunikation und/oder Energieübertragung mit dem Herzunterstützungssystem verbunden oder verbindbar ist, und wobei das System einen ersten Energiespeicher für eine Energieübertragung über die erste Spule an das Herzunterstützungssystem umfasst.

Unter einem Herzunterstützungssystem ist insbesondere ein System zur Unterstützung der Funktionalität eines Herzens eines Menschen oder Tieres zu verstehen, auch als Kunstherz, "ventricular assist device" (kurz "VAD") oder ventrikuläres Unterstützungssystem bezeichnet. Mit dem Ausdruck "extrakorporales Steuergerät" ist allgemein ein Steuergerät für ein Herzunterstützungssystem gemeint, welches nicht für eine Implantation in einen Körper eines Menschen oder Tieres vorgesehen, sondern für einen Einsatz außerhalb des Körpers vorgesehen und ausgebildet ist. Insbesondere umfasst das extrakorporale Steuergerät dazu eine Anzeige, beispielsweise ein Display, und/oder eine Eingabevorrichtung, insbesondere Tasten oder Knöpfe oder eine berührungssensitive Oberfläche. Unter einer drahtlosen Verbindung zwischen zwei Geräten oder zwischen einem System und einem Gerät ist insbesondere zu verstehen, dass die beiden Geräte beziehungsweise das System und das Gerät ausgebildet sind, über eine drahtlose bidirektionale Datenverbindung zu kommunizieren, insbesondere über eine Funkverbindung. Unter einer Ausbildung oder einer Einrichtung eines der Steuergeräte kann hier und im Folgenden verstanden werden, dass das Steuergerät entsprechend erforderliche Hardware-Komponenten aufweist, insbesondere einen Prozessor und Kommunikationsschnittstellen, und entsprechend programmiert ist, insbesondere der Prozessor. Beispielsweise kann es sich bei dem Steuergerät um ein handelsübliches Steuergerät für ein Herzunterstützungssystem handeln, welches erfindungsgemäß eingerichtet, insbesondere programmiert wird. Unter einem Kabel ist vor dem Hintergrund der Erfindung insbesondere ein als "Driveline" bezeichnetes Kabel zu verstehen, welches für eine Verbindung des Herzunterstützungssystems mit dem Steuergerät ausgebildet ist.

Das erfindungsgemäße System hat den Vorteil, dass das zweite extrakorporale Steuergerät eine zusätzliche Möglichkeit für den Benutzer bereitstellt, insbesondere den Patienten oder Arzt, Informationen über das Herzunterstützungssystem abzurufen und zu erfassen sowie das Herzunterstützungssystem anzusteuern, insbesondere einen Motor oder eine Pumpe des Herzunterstützungssystems. Insbesondere wenn das erste extrakorporale Steuergerät über das Kabel, also die oben genannte Driveline, mit dem Herzunterstützungssystem verbunden ist, ermöglicht das nicht körperlich verbundene zweite extrakorporale Steuergerät vorteilhafterweise eine einfache und schnelle Erfassung von Informationen und eine darauf abgestimmte Ansteuerung. Da das zweite Steuergerät physisch mit keiner anderen Vorrichtung verbunden sein muss, kann es vorteilhafterweise auch diskreter gehandhabt und verstaut werden. Ferner kann das zweite extrakorporale Steuergerät ausgebildet sein, für einen schnellen Zugriff über eine Halterung an einem Gürtel oder anderem Kleidungsstück des Patienten angebracht zu werden. Die flexible Handhabbarkeit über das zweite Steuergerät erlaubt vorteilhafterweise auch, das erste extrakorporale Steuergerät möglichst nahe oder unmittelbar am Körper zu tragen und somit den Abstand zum implantierten Herzunterstützungssystem für eine möglichst zuverlässige Interaktion zu verkürzen. Somit werden vorteilhafterweise einerseits die Sicherheit und Zuverlässigkeit und somit auch das Sicherheitsgefühl des Patienten erhöht, andererseits der Komfort und die Nutzerfreundlichkeit insgesamt verbessert. Darüber hinaus ist ein nicht physisch verbundenes Steuergerät unauffälliger in der Öffentlichkeit in Benutzungsreichweite mitzuführen und unauffälliger zu handhaben, so dass vorteilhafterweise dadurch die Verwendung eines Herzunterstützungssystems gegenüber Dritten weniger auffällig ist.

Vorzugsweise ist das zweite extrakorporale Steuergerät als redundante Einheit zum ersten extrakorporalen Steuergerät eingerichtet. Mit anderen Worten ist das zweite extrakorporale Steuergerät eingerichtet, bei einem Ausfall oder einer Fehlfunktion des ersten extrakorporalen Steuergeräts die Funktion des ersten extrakorporalen Steuergeräts zu übernehmen.

Gemäß einer besonders vorteilhaften Weiterbildung der Erfindung sind das erste Steuergerät und das zweite Steuergerät baugleich ausgeführt. Dies bedeutet insbesondere, dass das erste Steuergerät und das zweite Steuergerät die gleichen Hardware-Komponenten aufweisen und/oder einen gleichen strukturellen Aufbau aufweisen. Vorzugsweise weisen die beiden Steuergeräte auch die gleiche äußere Form und gleiche äußere Abmessungen aus. Dies hat den Vorteil, dass das erste Steuergerät bei Bedarf auf einfache Weise durch das zweite Steuergerät ersetzt werden kann, beispielsweise bei einer oben beschriebenen Fehlfunktion oder einem Ausfall des ersten Steuergeräts.

In einer besonders vorteilhaften Weiterbildung der Erfindung ist das erste Steuergerät als Server und das zweite Steuergerät als Client eingerichtet. Mit anderen Worten bilden die beiden extrakorporalen Steuergeräte ein Netzwerk, in welchem das erste extrakorporale Steuergerät die Rolle des Servers und das zweite extrakorporale Steuergerät die Rolle des Clients einnimmt. Damit kann vorteilhafterweise das für Netzwerke bewährte Client-Server-Modell eingesetzt werden.

Bevorzugt sind das erste Steuergerät und das zweite Steuergerät eingerichtet, bei einer Eingabe in das erste Steuergerät oder in das zweite Steuergerät das zweite Steuergerät beziehungsweise das erste Steuergerät zu veranlassen, dieselben Eingabe anzunehmen. Wenn die Eingabe Einstellungen in dem ersten beziehungsweise zweiten Steuergerät ändert, so werden dadurch vorteilhafterweise die gleichen Änderungen der Einstellungen in dem zweiten beziehungsweise ersten Steuergerät vorgenommen. Somit sind die beiden Steuergeräte vorteilhafterweise in einem gleichen oder synchronen Zustand.

In einer bevorzugten Weiterbildung der Erfindung ist der erste Energiespeicher vorzugsweise mit dem ersten Steuergerät insbesondere lösbar verbunden ist. Dadurch kann vorteilhafterweise über das erste extrakorporale Steuergerät eine Energieversorgung zum implantierten Herzunterstützungssystem geregelt werden. Vorzugsweise kann das System einen zweiten Energiespeicher aufweisen, welcher bevorzugt mit dem zweiten Steuergerät insbesondere lösbar verbunden ist. Dies hat den Vorteil, dass die Energieversorgung des Systems insgesamt noch besser abgesichert wird.

In einer weiteren vorteilhaften Ausgestaltung der Erfindung sind das erste Steuergerät und/oder das zweite Steuergerät eingerichtet, ein Alarmsignal auszugeben, wenn ein Mindestabstand zwischen den beiden Steuergeräten überschritten wird. Dabei können die Steuergeräte beispielsweise eingerichtet sein, den aktuellen Abstand über die Qualität der drahtlosen Funkverbindungen zwischen den beiden Steuergeräten abzuschätzen und mit dem Mindestabstand zu vergleichen. Als Maß für die Qualität kann beispielsweise eine Stärke oder Intensität der über die drahtlose Verbindung empfangenen Signale herangezogen werden. Wenn beispielsweise die Qualität, insbesondere die Signalstärke oder Signalintensität, weniger als ein vorgegebene Mindestwert beträgt, wird das Alarmsignal ausgelöst. Das Alarmsignal kann beispielsweise akustisch als Ton oder Tonfolge oder optisch über Leuchtelemente des Steuergeräts oder ein Display der Steuergeräte ausgegeben werden. Dies hat den Vorteil, dass der Benutzer oder Patient daran erinnert wird, beide Steuergeräte mit sich zu führen.

Gegenstand der Erfindung ist auch ein Herzunterstützungssystem umfassend ein erfindungsgemäßes System.

Zu den Vorteilen des erfindungsgemäßen Herzunterstützungssystems wird auf die oben ausgeführten korrespondierenden Vorteile des erfindungsgemäßen Systems verwiesen.

Ausführungsbeispiele der Erfindung sind in den Zeichnungen schematisch dargestellt und in der nachfolgenden Beschreibung näher erläutert. Für die in den verschiedenen Figuren dargestellten und ähnlich wirkenden Elemente werden gleiche Bezugszeichen verwendet, wobei auf eine wiederholte Beschreibung der Elemente verzichtet wird.

Es zeigen
- Figuren 1 und 2: ein Ausführungsbeispiel des erfindungsgemäßen Systems und
- Figur 3: ein Flussdiagramm eines Ausführungsbeispiels des erfindungsgemäßen Verfahrens.

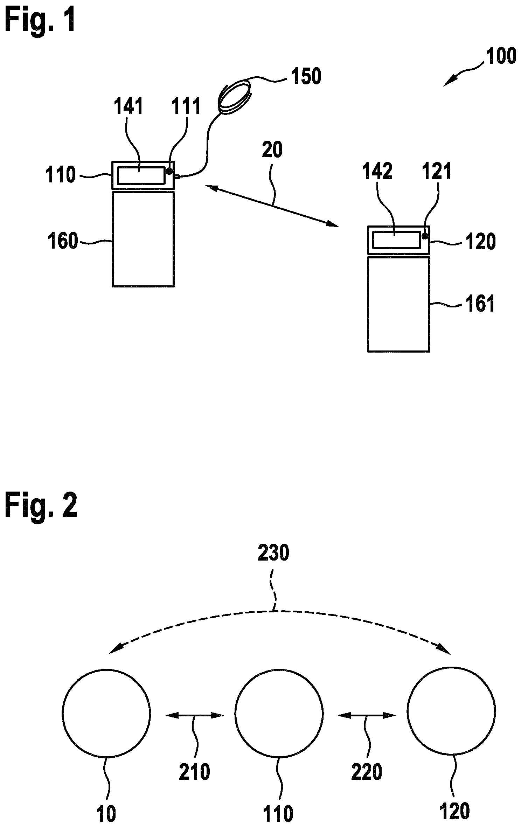

Figur 1 zeigt ein Ausführungsbeispiel des erfindungsgemäßen Systems 100. Das System 100 umfasst ein erstes extrakorporales Steuergerät 110 und ein zweites extrakorporales Steuergerät 120. Beide Steuergeräte weisen eine Schnittstelle 111, 121 für eine drahtlose Kommunikation 20 miteinander auf, beispielsweise eine Bluetooth^{®}-Schnittstelle 111, 121. Beispielsweise erfolgt die drahtlose Kommunikation über eine Funkkommunikation gemäß der MedRadio- oder MICS-Spezifikation (kurz für Medical Device Radiocommunications Service). Ferner können die beiden Steuergeräte 110, 120 eine integrierte Energiequelle aufweisen, beispielsweise eine Batterie oder einen Ackumulator. Dies ermöglicht eine Energieautarkie der Steuergeräte. Das erste Steuergerät 110 ist mit einer Spule 150 zur induktiven Energieübertragung an ein Herzunterstützungssystem verbunden. Ferner umfasst das erste Steuergerät 110 neben der möglichen integrierten Energiequelle eine weitere Energiequelle 160 für die Energieversorgung des Herzunterstützungssystems, beispielsweise eine Batterie oder einen Akkumulator. Die weitere Energiequelle 160 ist vorzugsweise lösbar mit dem ersten Steuergerät 110 verbunden, beispielsweise über einen Rast- oder Klickmechanismus. Da das zweite Steuergerät 120 an keine Vorrichtung physisch gebunden ist, zeichnet sich das zweite Steuergerät 120 durch eine hohe Benutzerfreundlichkeit aus. Für eine Anzeige von Information weisen die beiden Steuergeräte 110, 120 vorzugsweise ein Display 141, 142 auf, bevorzugt ein berührungssensitives Display 141, 142, welches sich auch zur Eingabe von Befehl in die Steuergeräte 110, 120 eignet.

In diesem Ausführungsbeispiel sind die beiden Steuergeräte 110, 120 baugleich ausgeführt und weisen bevorzugt einen gleichen strukturellen Aufbau auf, so dass das zweite Steuergerät 120 bei Bedarf die Funktion des ersten Steuergeräts übernehmen kann und somit als redundante Einheit zum ersten extrakorporalen Steuergerät vorgesehen ist. Insbesondere zu diesem Zweck kann auch das zweite Steuergerät 120 eine weitere Energiequelle 161 zur Absicherung der Energieversorgung des Herzunterstützungssystems umfassen.

In Figur 2 sind die Kommunikationswege 210, 220, 230 zwischen einem Herzunterstützungssystem 10, dem ersten extrakorporalen Steuergerät 110 und dem zweiten extrakorporalen Steuergerät 120 schematisch dargestellt. Das erste Steuergerät 110 ist eingerichtet, mit dem Herzunterstützungssystem 10 zu kommunizieren, entweder drahtlos, beispielsweise über Bluetooth^{®}, oder über eine Driveline. Beispielsweise werden zwischen dem ersten Steuergerät 110 und dem Herzunterstützungssystem 10 Informationen über Zustände wie beispielsweise Pumpendaten ausgetauscht. Ferner sind das erste Steuergerät 110 und das zweite Steuergerät 120 eingerichtet, drahtlos miteinander zu kommunizieren. Insbesondere sind sie eingerichtet, sich gegenseitig Änderungen in den Einstellungen und/oder weitere Statusinformation wie beispielsweise die Ladezustände der Energiespeicher 160, 161 zu übermitteln und anzupassen. Vorzugsweise ist das erste Steuergerät 110 dabei als Server und das zweite Steuergerät 120 als Client in einer Netzwerkkonfiguration eingerichtet. Somit kann das Herzunterstützungssystem 10 durch das zweite extrakorporale Steuergerät 120 vermittelt über das erste extrakorporale Steuergerät 110 angesteuert werden. In einer vorzugsweisen Ausgestaltung können ferner das Herzunterstützungssystem 10 und das zweite Steuergerät 120 für eine drahtlose Kommunikation 230 miteinander eingerichtet sein, was eine direkte Steuerung des Herzunterstützungssystems 10 durch das zweite Steuergerät 120 ermöglicht. Dies hat ferner den Vorteil, dass sowohl Informationen über das Herzunterstützungssystem 10 von beiden Steuergeräten 110, 120 direkt erhalten werden können, als auch beide Steuergeräte 110, 120 direkt das Herzunterstützungssystem 10 ansteuern können. Insbesondere kann der Benutzer oder Patient das Herzunterstützungssystem 10 nur über das zweite Steuergerät 120 direkt bedienen.

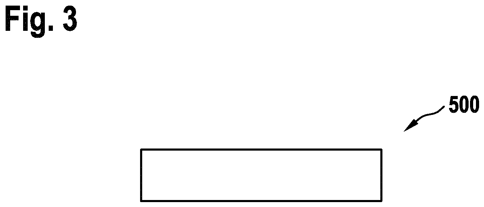

Figur 3 zeigt ein Blockdiagramm zu einem Ausführungsbeispiel des erfindungsgemäßen Verfahrens 500, welches beispielsweise mit einem Ausführungsbeispiel des erfindungsgemäßen Systems 100 wie oben beschrieben ausgeführt werden kann. Gemäß dem Ausführungsbeispiel 500 kann das Herzunterstützungssystems über ein erstes extrakorporales Steuergerät gesteuert werden. Ferner kann das Herzunterstützungssystems wie oben beschrieben über ein drahtlos mit dem ersten Steuergerät gekoppelten oder direkt über ein drahtlos mit dem Herzunterstützungssystem gekoppelten zweites extrakorporales Steuergerät gesteuert werden. Wenn das zweite Steuergerät drahtlos mit dem ersten Steuergerät verbunden ist, kann das Herzunterstützungssystem durch das zweite extrakorporale Steuergerät vermittelt durch das erste extrakorporale Steuergerät gesteuert werden.

## Patentansprüche

1. System (100) zur Steuerung eines Herzunterstützungssystems (10), umfassend ein erstes extrakorporales Steuergerät (110) und ein mit dem ersten Steuergerät (110) und/oder mit dem Herzunterstützungssystem (10) drahtlos verbundenes zweites extrakorporales Steuergerät (120), **dadurch gekennzeichnet, dass** das erste Steuergerät (110) mit einer ersten Spule (150) zur Kommunikation und/oder Energieübertragung mit dem Herzunterstützungssystem (10) verbunden oder verbindbar ist, wobei das System (100) einen ersten Energiespeicher (160) für eine Energieübertragung über die erste Spule (150) an das Herzunterstützungssystems (10) umfasst..

2. System (100) nach Anspruch 1, wobei das zweite extrakorporale Steuergerät (120) als redundante Einheit zum ersten extrakorporalen Steuergerät (110) eingerichtet ist.

3. System (100) nach einem der vorhergehenden Ansprüche, wobei das erste Steuergerät (110) als Server und das zweite Steuergerät (120) als Client eingerichtet ist.

4. System (100) nach einem der vorhergehenden Ansprüche, wobei das erste Steuergerät (110) und das zweite Steuergerät (120) baugleich ausgeführt sind.

5. System (100) nach einem der vorhergehenden Ansprüche, wobei der erste Energiespeicher (160) vorzugsweise mit dem ersten Steuergerät (110) insbesondere lösbar verbunden ist.

6. System (100) nach einem der vorhergehenden Ansprüche, wobei das System (100) einen zweiten Energiespeicher (161) aufweist, welcher vorzugsweise mit dem zweiten Steuergerät (120) insbesondere lösbar verbunden ist.

7. System (100) nach einem der vorhergehenden Ansprüche, wobei das erste Steuergerät (110) und das zweite Steuergerät (120) eingerichtet sind, bei einer Eingabe in das erste Steuergerät (110) oder in das zweite Steuergerät (120), wobei die Eingabe Einstellungen in dem ersten beziehungsweise zweiten Steuergerät (120) ändert, das zweite Steuergerät (120) beziehungsweise das erste Steuergerät (110) zu veranlassen, dieselben Änderungen der Einstellungen vorzunehmen.

8. System (100) nach einem der vorhergehenden Ansprüche, wobei das erste und/oder das zweite Steuergerät (110, 120) ein Display (141, 142) aufweisen, insbesondere ein berührungssensitives Display (141, 142) zur Eingabe von Befehlen.

9. System (100) nach einem der vorhergehenden Ansprüche, wobei das erste Steuergerät (110) und/oder das zweite Steuergerät (120) eingerichtet sind, ein Alarmsignal auszugeben, wenn ein Mindestabstand zwischen den beiden Steuergeräten (110, 120) überschritten wird.

10. Herzunterstützungssystem (10) umfassend ein System (100) nach einem der vorhergehenden Ansprüche.

## Claims

1. System (100) for controlling a cardiac support system (10), comprising a first extracorporeal control device (110) and a second extracorporeal control device (120) wirelessly connected to the first control device (110) and/or to the cardiac support system (10), **characterized in that** the first control device (110) is or can be connected, via a first coil (150), to the cardiac support system (10) for communication and/or energy transmission, the system (100) comprising a first energy store (160) for energy transmission via the first coil (150) to the cardiac support system (10).

2. System (100) according to claim 1, wherein the second extracorporeal control device (120) is configured as a redundant unit to the first extracorporeal control device (110).

3. System (100) according to either of the preceding claims, wherein the first control device (110) is configured as a server and the second control device (120) is configured as a client.

4. System (100) according to any of the preceding claims, wherein the first control unit (110) and the second control unit (120) are structurally identical.

5. System (100) according to any of the preceding claims, wherein the first energy store (160) is preferably connected, in particular detachably connected, to the first control device (110).

6. System (100) according to any of the preceding claims, wherein the system (100) has a second energy store (161) which is preferably connected, in particular detachably connected, to the second control device (120).

7. System (100) according to any of the preceding claims, wherein the first control device (110) and the second control device (120) are configured, when an input is made to the first control device (110) or to the second control device (120), the input changing settings in the first or second control device (120), respectively, to cause the second control device (120) or the first control device (110), respectively, to make the same changes to the settings.

8. System (100) according to any of the preceding claims, wherein the first and/or the second control device (110, 120) have a display (141, 142), in particular a touch-sensitive display (141, 142) for inputting commands.

9. System (100) according to any of the preceding claims, wherein the first control device (110) and/or the second control device (120) are configured to output an alarm signal when a minimum distance between the two control devices (110, 120) is exceeded.

10. Cardiac support system (10) comprising a system (100) according to any of the preceding claims.

## Revendications

1. Système (100) permettant la commande d'un système d'assistance cardiaque (10), comprenant un premier appareil de commande (110) extracorporel et un second appareil de commande (120) extracorporel connecté sans fil au premier appareil de commande (110) et/ou au système d'assistance cardiaque (10), **caractérisé en ce que** le premier appareil de commande (110) est connecté ou peut être connecté à une première bobine (150) pour la communication et/ou la transmission d'énergie avec le système d'assistance cardiaque (10), dans lequel le système (100) comprend un premier accumulateur d'énergie (160) pour une transmission d'énergie par l'intermédiaire de la première bobine (150) au système d'assistance cardiaque (10).

2. Système (100) selon la revendication 1, dans lequel le second appareil de commande (120) extracorporel est conçu en tant qu'unité redondante par rapport au premier appareil de commande (110) extracorporel.

3. Système (100) selon l'une des revendications précédentes, dans lequel le premier appareil de commande (110) est conçu en tant que serveur et le second appareil de commande (120) est conçu en tant que client.

4. Système (100) selon l'une des revendications précédentes, dans lequel le premier appareil de commande (110) et le second appareil de commande (120) sont réalisés de manière identique.

5. Système (100) selon l'une des revendications précédentes, dans lequel le premier accumulateur d'énergie (160) est de préférence connecté, en particulier de manière amovible, au premier appareil de commande (110).

6. Système (100) selon l'une des revendications précédentes, dans lequel le système (100) présente un second accumulateur d'énergie (161) qui est de préférence connecté, en particulier de manière amovible, au second appareil de commande (120).

7. Système (100) selon l'une des revendications précédentes, dans lequel le premier appareil de commande (110) et le second appareil de commande (120) sont configurés pour, lors d'une saisie dans le premier appareil de commande (110) ou dans le second appareil de commande (120), dans lequel la saisie modifie des réglages dans le premier ou le second appareil de commande (120), amener le second appareil de commande (120) ou le premier appareil de commande (110) à effectuer les mêmes modifications des réglages.

8. Système (100) selon l'une des revendications précédentes, dans lequel le premier et/ou le second appareil de commande (110, 120) présentent un écran (141, 142), en particulier un écran (141, 142) tactile pour la saisie d'instructions.

9. Système (100) selon l'une des revendications précédentes, dans lequel le premier appareil de commande (110) et/ou le second appareil de commande (120) sont configurés pour émettre un signal d'alarme lorsqu'une distance minimale entre les deux appareils de commande (110, 120) est dépassée.

10. Système d'assistance cardiaque (10) comprenant un système (100) selon l'une des revendications précédentes.
